# EUROPÄISCHE PATENTANMELDUNG

(11) **EP 0 643 067 A1**
(43) Veröffentlichungstag der Anmeldung: **15.03.1995**
(21) Anmeldenummer: 94113986.7
(22) Anmeldetag: 07.09.1994
(51) Int. Cl.: C07H 9/04

(54) **Verfahren zur Herstellung von Zuckeracetoniden**

(30) Priorität: 10.09.1993 DE 4330701
(71) Anmelder: BOEHRINGER INGELHEIM KG, D-55216 Ingelheim (DE); BOEHRINGER INGELHEIM INTERNATIONAL GmbH, D-55216 Ingelheim am Rhein (DE)
(72) Erfinder: Sobotta, Rainer Dr. Dipl. Chem., D-55218 Ingelheim am Rhein (DE); Klingler, Franz Dietrich Dr.-Ing., D-64347 Griesheim (DE); Schneider, Heinrich Dr. -Chem., D-55218 Ingelheim am Rhein (DE)

(57) **Zusammenfassung**

Die vorliegende Erfindung betrifft ein neuartiges Verfahren zur Herstellung von Zuckeracetoniden - wie z. B. von 1,2:5,6-Di-O-isopropy-liden-α-D-glucofuranose - durch Umsetzung des entsprechenden Zuckers - beispielsweise D-(+)-Glucose - mit Aceton 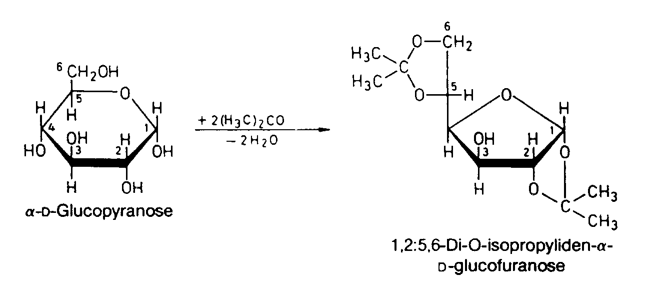
in Gegenwart einer Lewissäure und einem Carbonsäurederivat der allgemeinen Formel 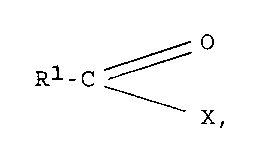
worin X eine durch eine Hydroxylgruppe substituierbare Austrittsgruppe verkörpert.

## Beschreibung

Die vorliegende Erfindung betrifft ein verbessertes Verfahren zur Herstellung von Zuckeracetoniden - wie z. B. der 1,2-5,6-Diaceton-D-glucose (1,2:5,6-Di-O-isopropyliden-α-D-glucofuranose) 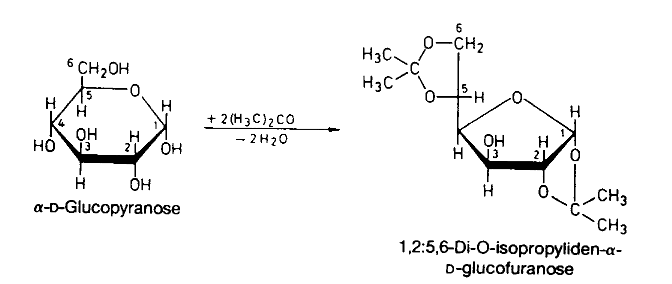
aus dem entsprechenden Zuckern - im Beispiel D-Glucose - und Aceton in Gegenwart von Verbindungen der allgemeinen Formel: 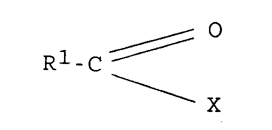
worin X eine gegen eine Hydroxygruppe substituierbare Austrittsgruppe und bevorzugt 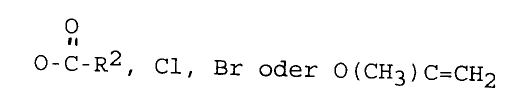
und R¹ und R² unabhängig voneinander Methyl, Ethyl, Propyl, iso-Propyl, Butyl - verzweigt oder unverzweigt, Pentyl - verzweigt oder unverzweigt sowie Hexyl - verzweigt oder unverzweigt - bedeuten können. Als Ausgangsstoffe sind im Sinne der vorliegenden Erfindung in erster Linie Monosaccharide - wie z. B. D-Galactose, D-Glucose, L- sowie D-Arabinose, Fructose D-Mannose, Sorbose, D-Xylose, D-Ribose, D-Mannit oder L-Ascorbinsäure - oder auch Oligosaccharide oder geeignete Polysaccharide zu verstehen sofern sie die Bedingung erfüllen, daß sie zwei sterisch benachbarte cis-ständige Hydroxylgruppen aufweisen.

Diisopropyliden-Derivate der Zucker - wie z. B. 1,2-5,6-Diaceton-D-glucose stellen u. a. zentrale Zwischenprodukte für zahlreiche andere Zuckerderivate Glucoseabkömmlinge dar, die u.a. als Arzneimittel große Bedeutung haben. Als Beispiele seien lediglich das 2-Deoxy-D-riboseanilid oder Amiprilose genannt.

Daneben kann 1,2-5,6-Diaceton-D-glucose als chiraler Ligand in Komplexen eingesetzt werden, die enantioselektive Reaktionen ermöglichen [F.D. Klingler und M. Psiorz, Chimicaoggi 1992, 47].

Diese zentrale Rolle der 1,2-5,6-Diaceton-D-glucose ist dafür verantwortlich, daß ein jährlicher Bedarf in Tonnengrößenordnungen an diesem Zwischenprodukt besteht. Aus dem Stand der Technik ist allgemein bekannt, daß Monosaccharide, die zwei sterisch benachbarte, cis-ständige OH-Gruppen enthalten, mit Aldehyden oder Ketonen in Gegenwart von Schwefelsäure, Zinkchlorid oder Phosphor(V)-oxid zu den entsprechenden Acetalen umgesetzt werden können (E. Fischer, 1895).

So ist z. B. die 1,2:5,6-Di-O-isopropyliden-α-D-glucofuranose (Diaceton-α-D-glucose) durch Umsetzung von D-Glucose mit Aceton in Gegenwart von Schwefelsäure erhältlich. Dabei muß - zur Erzielung hoher Umsätze - das aus der Ketalisierung resultierende Wasser gebunden bzw. aus dem Reaktionsgemisch entfernt werden.

Des weiteren ist aus dem Stand der Technik die Herstellung von Diacetonglucose in Gegenwart sauer reagierender Katalysatoren, wie Lewis-Säuren, beispielsweise komplexe Verbindungen des Bortrifluorids, Aluminiumhalogenide - wie zum Beispiel Aluminium(III)-chlorid oder -bromid -, Zinnsalze oder Halogenide seltener Erden bekannt.

Als weitere Katalysatoren sind aus dem Stand der Technik u.a. auch Jod, Gips oder Molekularsiebe bekannt. Jedoch ist die Verwendung der bislang als geeignet bekannten Katalysatoren - nicht nur hinsichtlich der Reaktionen im industriellen Maßstab - mit gravierenden Nachteilen verbunden, von denen beispielhaft die folgenden genannt sein sollen:
- bei der Verwendung von Mineralsäuren oder Phosphorpentoxid müssen große Mengen dieser Agenzien eingesetzt werden, was zum einen nur einen geringen Durchsatz erlaubt und zum anderen große Entsorgungsprobleme der aus der anschließend notwendigen Neutralisation resultierenden Salze mit sich bringt;
- bei der Verwendung von Jod sind große Mengen Lösungsmittel erforderlich, die ebenfalls nur einen geringen Durchsatz erlauben;
- beim Einsatz eines zusätzlichen Lösungsmittels, welches in der Lage ist, ein Azeotrop mit Wasser zu bilden, wird eine weitere Vergrößerung des Volumens des Reaktorgefäßes erforderlich - zudem geht der Einsatz eines Schleppmittels - wie beispielsweise Pentan - mit einer Siedepunktserniedrigung einher, womit die Reaktionstemperatur limitiert wird und womit sich die Reaktionszeit entsprechend verlängert;
- der Einsatz fester Katalysatoren bereitet durch die ebenfalls stattfindenden Karamelisierungsreaktionen ebenfalls Schwierigkeiten - außerdem ist beispielsweise die Wiederaufbereitung von Ionenaustauschern mit einem sehr großen Aufwand verbunden;
- daneben haftet vielen Umsetzungen der Nachteil an, daß durch die - in ihrem Ausmaß von den jeweiligen Reaktionsbedingungen abhängigen - Nebenreaktionen - wie z.B. die Selbstkondensation des Acetons - z.T. teerartige Nebenprodukte entstehen, die einerseits die Wirksamkeit des Katalysators beeinträchtigen und andererseits zu einer unerwünschten Verunreinigung des Reaktionsproduktes führen, die teilweise lediglich im Rahmen einer chromatographischen Reinigung wieder entfernbar sind.

Die Aufgabe der vorliegenden Erfindung besteht somit darin, ein Verfahren zur Herstellung von Zuckeracetoniden zur Verfügung zu stellen, das die aus dem Stand der Technik bekannten Nachteile nicht beinhaltet und welches die gewünschten Diisopropyliden-Derivate - wie z. B. 1,2-5,6-Diaceton-D-glucose oder Diaceton-D-mannose in hoher Ausbeute verfügbar macht.

Erfindungsgemäß wird diese Augabe dadurch gelöst, daß man den Zucker in Gegenwart einer Lewis-Säure - vorzugsweise Bortrifluorid-Etherat - und einem Carbonsäurederivat der allgemeinen Formel 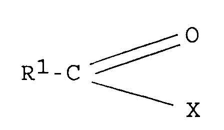
worin X eine durch eine Hydroxylgruppe substituierbare Austrittsgruppe und bevorzugt

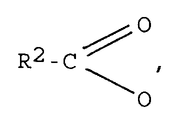

Cl, Br oder O-(CH₃)C=CH₂ und R¹ und R² unabhängig voneinander Methyl, Ethyl, Propyl, iso-Propyl, Butyl - verzweigt oder unverzweigt, Pentyl - verzweigt oder unverzweigt - sowie Hexyl - verzweigt oder unverzweigt - bedeuten können,
mit bzw. in Aceton umsetzt.

Das erfindungsgemäße Herstellungsverfahren besitzt gegenüber den aus dem Stand der Technik bekannten Verfahren denjenigen Vorteil, daß keine schwierig zu entsorgenden Abfallprodukte anfallen. Ferner kommt das erfindungsgemäße Verfahren mit einem relativ kleinen Lösungsmittelvolumen aus.

Zur Durchführung dieses Verfahrens wird der - wasserfreie - Zucker in dem Reaktionsgefäß vorgelegt, in Aceton suspendiert und mit dem Carbonsäurederivat sowie mit einer Lewis-Säure versetzt. Geeignete Lewis-Säuren sind aus dem Stand der Technik bekannt - zu ihnen zählen beispielsweise komplexe Verbindungen des Bortrifluorids, Aluminiumhalogenide, Zinnsalze oder Halogenide seltener Erden. Bevorzugt wird Bortrifluorid-Etherat als Lewis-Säure eingesetzt. Danach wird die Reaktionslösung auf eine Temperatur im Bereich von 40 bis 58°C - vorzugsweise auf Rückflußtemperatur (bei der Verwendung von Acetanhydrid ca. 58° C) über einen Zeitraum von 4 bis 8 Stunden - vorzugsweise 6 Stunden - erwärmt, wobei eine farblose Reaktionslösung entsteht. Nach beendeter Reaktion wird das Reaktionsgemisch abgekühlt und unter Rühren und Kühlen mit einer verdünnten wässerigen Lösung einer basisch reagierenden Verbindung eines Alkali- oder Erdalkalimetalls - wie z. B. mit einer verdünnten wässerigen Lösung eines Alkali- oder Erdalkalimetallhydroxids - vorzugsweise Natriumhydroxyd - versetzt.

Danach wird das nicht umgesetzte Aceton - vorzugsweise unter vermindertem Druck - auf destillativem Wege entfernt und der Destillationssumpf mit einem unter den beschriebenen Reaktionsbdingungen inerten Extraktionsmittel - wie beispielsweise einem aliphatischen oder aromatischen Kohlenwasserstoff - vorzugsweise Toluol - versetzt.

Die wässerige Phase wird erschöpfend extrahiert - und aus den vereinigten Extrakten wird, ggf. unter Zugabe eines Fällungsmittels - vorzugsweise Cyclohexan -, bei erhöhter Temperatur, die z.B. im Bereich von 50 bis 60 - vorzugsweise im einem Bereich von 50 - 55°C liegt, und ggf. unter Kühlung die resultieren Diacetonzucker ausgefällt. Die ausgefallenen Kristalle werden isoliert und ggf. mit einem geeigneten Lösungsmittel - vorzugsweise gekühltem Cyclohexan - gewaschen und anschließend bei erhöhter Temperatur - vorzugweise bei 40°C - im Umlufttrockenschrank bis zur Gewichtskonstanz getrocknet.

Die eingangs genannten Aufgaben werden durch die in den folgenden Beispielen beschriebenen Verfahren gelöst. Verschiedenartige, andere Ausgestaltungen des Verfahrens werden für den Fachmann aus der vorliegenden Beschreibung ersichtlich. Es wird jedoch ausdrücklich darauf hingewiesen, daß das Beispiel und die diesem zugeordnete Beschreibung lediglich zum Zweck der Erläuterung und Beschreibung vorgesehen und nicht als Einschränkung der Erfindung anzusehen sind. Insbesondere wird darauf hingewiesen, daß die in den Beispielen zur Herstellung von 1,2:5,6-Di-O-isopropyliden-α-D-glucofuranose und Diaceton-D-mannose beschriebene Synthesefolgen auch auf die Herstellung anderer Isopropyliden-Zucker übertragen werden kann. Als in Frage kommende Ausgangsstoffe seien beispielsweise folgende weitere Monosaccharide genannt: D-Galactose, L- sowie D-Arabinose, Fructose, Sorbose, D-Xylose, D-Ribose, D-Mannit oder L-Ascorbinsäure.

### Beispiel 1

In ein ausgetrocknetes Reaktionsgefäß werden unter Inertgas 3,78 kg (20,98 mol) wasserfreie D-(+)-Glucose eingetragen und in 74 l Aceton suspendiert. Danach werden 4,28 kg (41,92 mol) Essigsäureanhydrid und 31 ml Bortrifluorid-Etherat zugefügt. Anschließend wird die Reaktionsmischung über einen Zeitraum von 6 Stunden auf 58° C Rückflußtemperatur erwärmt. Danach wird die nunmehr klare, farblose Reaktionsmischung auf Raumtemperatur (ca. 20° C) abgekühlt. Die so behandelte Reaktionsmischung wird in eine Lösung von 3.36 kg Natriumhydroxid in 16.8 l entmineralisierten Wasser eingetragen, wobei die Temperatur des Reaktionsgemisches mittels Kühlung unterhalb von 35° C gehalten wird. Aus der Reaktionsmischung wird das Aceton bzw. die leicht flüchtigen Bestandteile der Reaktionsmischung unter schwachem Vakuum bei einer Sumpftempertur von ca. 45° C abdestilliert.

Nach erfolgter Destillation wird der Destillationsrückstand erschöpfend mit Toluol extrahiert.

Die vereinigten organischen Extrakte (ca. 24 l) werden im Vakuum auf ca. 9 l eingeengt und mit 14,5 l Cyclohexan versetzt und ca. 15 Minuten bei einer Temperatur im Bereich von 50 - 55° C gerührt, wobei eine klare gelbe Lösung entsteht.

Danach wird eine Stunde bei einer Temperatur von ca. 40° C nachgerührt, wobei Kristallisation eintritt. Die Kristallsuspension wird auf ca. 10° C abgekühlt und danach ca. 1,5 Stunden bei einer Temperatur im Bereich von 2 - 4° C gerührt.

Der resultierende Kristallbrei wird über ein Füllraumfilter filtriert und der Filterkuchen dreimal mit jeweils 400 ml gekühltem Cyclohexan gewaschen. Der verbliebene Rückstand wird anschließend im Umlufttrockenschrank bei 40° C getrocknet. Man erhält auf diese Weise 2,956 kg (53 % d. Th.) der 1,2 - 5,6-Diaceton-D-glucose.

### Beispiel 2

In ein ausgetrocknetes Reaktionsgefäß werden unter Inertgas 3,00 kg wasserfreie D-(+)-Mannose eingetragen und in 42 l Aceton suspendiert. Danach werden 28 g Bortrifluorid-Etherat zugefügt. Anschließend wird die Reaktionsmischung auf 58°C Rückflußtemperatur erwärmt und innerhalb eines Zeitintervalls von 1 Stunde mit 2,5 kg Essigsäureanhydrid versetzt. Danach wird das Reaktionsgemisch über einen Zeitraum von 5 Stunden auf Rückflußtemperatur erhitzt und ungefähr die Hälfte des ursprünglich eingesetzten Acetons abdestilliert. Die nunmehr klare, farblose Reaktionsmischung auf eine Temperatur von 10°C abgekühlt und danach mit 12,3 l 25%-iger Natronlauge versetzt, wobei der pH-Wert des Reaktionsgemisches nach der Zugabe mindestens 8 betragen soll. Aus der Reaktionsmischung wird das Aceton bzw. die leicht flüchtigen Bestandteile der Reaktionsmischung unter schwachem Vakuum abdestilliert.

Nach erfolgter Destillation wird der Destillationsrückstand bei einer Temperatur von 65°C mit Toluol versetzt und Abtrennung der organischen Phase mit Toluol extrahiert.

Die vereinigten organischen Extrakte werden im Vakuum eingeengt und bei einer Temperatur von ca. 70°C über ein beheiztes Druckfilter filtriert. Anschließend wird der Filtrationsrückstand zweimal mit je 0,5 l Toluol gewaschen. Beim anschließenden Abkühlen tritt bei ca. 50°C der Kristallisationsbeginn ein. Nach dem Abkühlen auf eine Temperatur im Bereich von 5 bis 7°C entsteht ein Kristallbrei, der abgenutscht wird. Das Nutschengut wird danach im Vakuum- oder Umlufttrockenschrank bei einer Temperatur in einem Intervall von 40-45°C getrocknet.

Auf diese Weise werden 4,40 kg (67.7 % t.q. bezogen auf die eingesetzte Mannose) Diaceton-D-mannose erhalten.

## Patentansprüche

1. Verfahren zur Herstellung von Zuckeracetoniden aus dem entsprechenden Zucker und Aceton in Gegenwart einer Lewissäure, dadurch gekennzeichnet, daß man die Umsetzung in Gegenwart eines Carbonsäurederivates folgender allgemeiner Formel 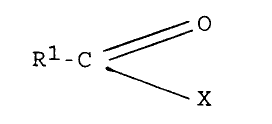 worin X eine durch eine Hydroxylgruppe substituierbare Austrittsgruppe und R¹ Methyl, Ethyl, Propyl, iso-Propyl, Butyl - verzweigt oder unverzweigt -, Pentyl - verzweigt oder unverzweigt - sowie Hexyl - verzweigt oder unverzweigt - bedeuten, bei einer Temperatur im Bereich von 40 bis 58° C durchführt, die Reaktionsmischung nach erfolgter Umsetzung abkühlt und mit einer verdünnten wässerigen Lösung einer basisch reagierenden Verbindung eines Alkali- oder Erdalkalimetalls in Kontakt bringt, das resultierende Gemisch mit einem aliphatischen oder aromatischen Kohlenwasserstoff extrahiert, die vereinigten Extrakte auf eine Temperatur im Bereich von 50 - 60°C erwärmt ggf. filtriert und kristallisiert oder mit einem Fällungsmittel versetzt und Di-isopropyliden-Zucker ausfällt, die ausgefallenen Kristalle isoliert, mit einem geeigneten Lösungsmittel wäscht und von Lösungsmittelresten befreit.

2. Verfahren nach Anspruch 1, dadurch gekennzeichnet, daß man die Umsetzung in Gegenwart eines Carbonsäurederivates folgende allgemeine Formel 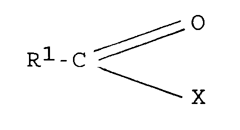 worin X die Bedeutung von 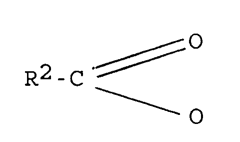 Cl, Br oder -O-(CH₃)C=CH₂ und R¹ und R² unabhängig voneinander Methyl, Ethyl, Propyl, iso-Propyl, Butyl - verzweigt oder unverzweigt, Pentyl - verzweigt oder unverzweigt oder Hexyl - verzweigt oder unverzweigt haben können, unter Rückflußbedingungen durchführt, die Reaktionsmischung nach erfolgter Umsetzung abkühlt und in die verdünnte wässerige Lösung eins Alkali- oder Erdalkalihydroxids - vorzugsweise in verdünnte Natronlauge einträgt, das resultierende Gemisch mit Toluol extrahiert, die vereinigten Extrakte auf eine Temperatur im Bereich von 50 bis 55° C erwärmt und ggf. mit Cyclohexan als Fällungsmittel versetzt, die Lösung abkühlt und die dabei ausfallenden Kristalle der 1,2-5,6-Diaceton-D-glucose isoliert, mit gekühltem Cyclohexan wäscht und abschließend trocknet.

3. Verfahren nach Anspruch 2, dadurch gekennzeichnet, daß als Carbonsäurederivat Acetanhydrid eingesetzt wird.

4. Verfahren nach einem der Ansprüche 1 bis 3, dadurch gekennzeichnet, daß man ein Monosaccharid, das zwei sterisch benachbarte cis-ständige Hydroxylgruppen besitzt, als Zucker einsetzt.

5. Verfahren nach Anspruch 4, dadurch gekennzeichnet, daß man als Monosaccharid D-Galactose, L-Arabinose, D-Arabinose, Fructose, D-Mannose, Sorbose, D-Xylose, D-Ribose, D-Mannit oder L-Ascorbinsäure einsetzt.

6. Verfahren nach einem der Ansprüche 1 bis 3, dadurch gekennzeichnet, daß man ein Disaccharid, das zwei sterisch benachbarte cis-ständige Hydroxylgruppen besitzt, einsetzt.

7. Verfahren nach einem der Ansprüche 1 bis 3, dadurch gekennzeichnet, daß man als Zucker ein Polysaccharid, das zwei sterisch benachbarte Hydroxylgruppen besitzt, einsetzt.

8. Verfahren zur Herstellung von 1,2:5,6-Di-O-isopropyliden-α-D-glucofuranose aus dem entsprechenden Zucker und Aceton in Gegenwart einer Lewissäure nach einem der Ansprüche 1 bis 3, dadurch gekennzeichnet, daß man D-Glucose in Gegenwart eines Carbonsäurederivates folgender allgemeiner Formel 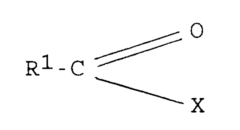 worin X eine durch eine Hydroxylgruppe substituierbare Austrittsgruppe und R¹ Methyl, Ethyl, Propyl, iso-Propyl, Butyl - verzweigt oder unverzweigt -, Pentyl - verzweigt oder unverzweigt - sowie Hexyl - verzweigt oder unverzweigt - bedeuten, bei einer Temperatur im Bereich von 40 bis 58° C mit Aceton, die Reaktionsmischung nach erfolgter Umsetzung abkühlt und mit einer reagierenden Verbindung eines Alkali- oder Erdalkalimetalls in Kontakt bringt, das resultierende Gemisch mit einem aliphatischen oder aromatischen Kohlenwasserstoff extrahiert, die vereinigten Extrakte auf eine Temperatur im Bereich von 50 - 60°C erwärmt und mit einem Fällungsmittel versetzt und die 1,2-5,6-Diaceton-D-glucose ausfällt, die ausgefallenen Kristalle isoliert, mit einem geeigneten Lösungsmittel wäscht und von Lösungsmittelresten befreit.

9. Verfahren nach Anspruch 8, dadurch gekennzeichnet, daß man die Umsetzung in Gegenwart eines Carbonsäurederivates folgende allgemeine Formel 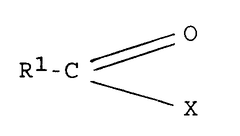 worin X die Bedeutung von 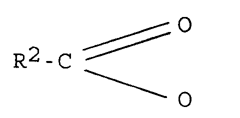 Cl, Br oder -O-(CH₃)C=CH₂ und R¹ und R² unabhängig voneinander Methyl, Ethyl, Propyl, iso-Propyl, Butyl - verzweigt oder unverzweigt, Pentyl - verzweigt oder unverzweigt oder Hexyl - verzweigt oder unverzweigt haben können, unter Rückflußbedingungen durchführt, die Reaktionsmischung nach erfolgter Umsetzung abkühlt und in verdünnte Natronlauge einträgt, das resultierende Gemisch mit Toluol extrahiert, die vereinigten Extrakte auf eine Temperatur im Bereich von 50 bis 55° C erwärmt und mit Cyclohexan versetzt, die Lösung abkühlt und die dabei ausfallenden Kristalle der 1,2-5,6-Diaceton-D-glucose isoliert, mit gekühltem Cyclohexan wäscht und abschließend trocknet.

10. Verfahren nach Anspruch 8, dadurch gekennzeichnet, daß als Carbonsäurederivat Acetanhydrid eingesetzt wird.
